# EUROPEAN PATENT APPLICATION

(11) **EP 2 096 438 A1**
(43) Date of publication of application: **02.09.2009**
(21) Application number: 08450024.8
(22) Date of filing: 28.02.2008
(51) Int. Cl.: G01N 33/564, G01N 33/574, G01N 33/569, C07K 16/14, A61P 35/00, A61P 31/00, A61P 37/00

(54) **Methods for prognosing the status of patients**

(71) Applicant: Thurnher, Martin, 6020 Innsbruck (AT)
(72) Inventor: Thurnher, Martin, 6020 Innsbruck (AT)
(74) Representative: Sonn & Partner Patentanwälte

(57) **Abstract**

The invention discloses a method for prognosing the status of a tumor patient, an autoimmune patient, a patient suffering from an infectious disease or a transplantation patient, **characterised in that** the level of antibodies against TH17 immune response eliciting microorganisms is determined in said patient, and prognosing the status of the patient upon the level of these antibodies determined in the patient by determining a better status for a patient with a lower level of these antibodies compared to the average level or by determining a worse status for a patient with a higher level of these antibodies compared to the average level.

## Description

Renal cell carcinoma (RCC), which arises from the renal epithelium, is the major histologic subtype of human kidney cancer and accounts for 85% of renal cancers and for 2-3% of all cancers in adults. Approximately 30.000 to 40.000 new cases of RCC are diagnosed in the United States each year resulting in more than 12.000 deaths per year. At diagnosis a quarter of RCC patients present with advanced disease and one third of the patients with organ-confined tumors at the time of resection will develop metastatic disease. Median survival for patients with metastatic disease is about 13 months.

The physiological drug resistance of renal epithelial cells may be responsible for the low response rates of RCC to chemotherapy. Instead frequent tumor infiltration by leukocytes, occasional spontaneous tumor regression and tumor regression that may occur in the context of cytokine therapy support the value of immunotherapy including therapeutic anti-tumor vaccination. Since RCC is a highly vascularized tumor, the pathways of angiogenesis are additional targets of RCC therapy.

Defining the prognosis of RCC is important for both therapeutic decision-making and counselling patients. Several prognostic factors that correlate with overall survival in patients with metastatic RCC have previously been identified. These factors include Karnofsky performance status, serum lactate dehydrogenase (LDH), corrected serum calcium, haemoglobin, Fuhrman nuclear grade and serum cytokines. In addition, recently interleukin-4 promoter polymorphisms were described as a genetic prognostic factor for survival in metastatic RCC.

A recent Italian case-control study by Bravi et al. reported that a diet rich in bread and refined cereals may have an unfavourable influence on RCC (Bravi et al, 2007). Among several possibilities, one interpretation of the finding by Bravi et al. is that the unfavourable influence of bread on RCC reflects an underlying intolerance of wheat, gluten-containing cereals or other bread ingredients. Broken immune tolerance can cause inflammatory and immune responses to food antigens. Intolerance to wheat gluten, for instance, can lead to the development of celiac disease. Gluten-derived peptides presented on distinct MHC class II initiate CD4+ T helper (TH) cell responses followed by B cell activation and antibody production. In addition to autoantibodies against the enzyme tissue transglutaminase (TG2), IgG and IgA antibodies against gliadin, the alcohol-soluble protein fraction of gluten, are disease-specific markers.

The knowledge of the status or prognosis of a tumor patient is detrimental for the specific definition and set-up for the treatment. Patients with a good prognosis receive a less stringent treatment; patients with a poor prognosis have to receive a more stringent therapy with sometimes life threatening doses of medicaments.

Also for other diseases, such as autoimmune diseases or infectious diseases, or for organ transplant patients, markers for the prognosis or status of the disease is important for defining the treatment regimen.

It is therefore an object of the present invention to provide markers for the status and/or prognosis of a given patient, especially a tumor patient, an autoimmune patient, a patient suffering from an infectious disease or a transplantation patient.

Therefore, the present invention provides a method for prognosing the status of a tumor patient, an autoimmune patient, a patient suffering from an infectious disease or a transplantation patient, **characterised in that** the level of antibodies against TH17 immune response eliciting microorganisms is determined in said patient, and prognosing the status of the patient upon the level of these antibodies determined in the patient by determining a better status for a patient with a lower level of these antibodies compared to the average level or by determining a worse status for a patient with a higher level of these antibodies compared to the average level.

During the research for the present invention it surprisingly turned out that antibodies against TH17 immune response eliciting microorganisms, such as Saccharomyces cerevisiae, Candida albicans or Klebsiella pneumoniae, directly correlate to the prognosis/status of a patient, i.e. the lower the titer of these antibodies is, the better the status or prognosis of the patient.

This was even more surprising in view of the teachings of Bravi et al. according to which intolerance of wheat is the reason for unfavourable influence of bread on RCC. In fact, the present inventors initially examined the possible relationship between humoral immune responses against bread components and an unfavourable clinical course of RCC. For this purpose, an ELISA-based screening for food-specific IgG in sera of patients with metastatic RCC was performed. During this research, however, it turned out that RCC patients with elevated serum levels of IgG antibodies against S. cerevisiae, commonly known as baker's yeast and yet another bread component, have an unfavourable clinical course. It could be shown that serum levels of IgG against S. cerevisiae predicts survival in patients with metastatic RCC. The data therefore confirmed not cereals but baker's yeast being the critical component of bread, that may cause immune deviation and impaired immunosurveillance in predisposed RCC patients. Since these kind of antibodies seem to be strikingly characteristic for a TH17 differentiation, it is clear that enhanced TH17 development is not only relevant for RCC, but also for all other tumor diseases, solid and hematological cancers.

Although it has been known that humoral immune responses against food components can also amount to an increased production of IgG against S. cerevisiae, commonly known as baker's or brewer's yeast, relevance of such common antibodies for e.g. tumor patients has been completely unknown. Unusually high levels of serum IgG against S. cerevisiae have been reported to be associated with inflammatory bowel diseases and in particular with Crohn's disease. Serum IgG against S. cerevisiae have also been detected in patients with celiac disease or patients with ulcerative colitis (Barta et al., 2003).

In view of the TH17 background, it is clear that the present invention is also relevant for infectious diseases and autoimmune diseases, as well as for organ transplant rejection, because it is known that Th17 cells play an important role in these diseases.

More in detail, the present invention relates to a method for prognosing the status of a tumor patient, wherein the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level. For example, if detected by ELISA, serum levels (OD) of 0.074 - 1.158 (median 0.200) were measured for Saccharomyces IgG. The higher this level is, the worse the prognosis. Although there is no threshold value from which "poor prognosis" status, any level above the median is in any way bad and could e.g. exclude the patient from immune therapy, so that e.g. only "targeted therapy" with tyrosine kinase inhibitors is suited.

Although antibody detection in blood-derived patient samples (blood, serum, plasma, etc.) is preferred, the antibodies may also be detected in other antibody containing body fluids or tissue (biopsy material, etc.).

The present invention also relates to a method for prognosing the status of a patient suffering from an autoimmune disease, **characterised in that** the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level. The determined level of antibodies will then have direct implications on the intensity of the therapy and can be used as a marker for the course and development of the treatment, or for the efficiency thereof.

The present invention also relates to a method for prognosing the status of a patient suffering from an infectious disease, wherein the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level. The antibodies determined according to the present invention display the immune status of a patient or even a healthy person, thereby indicating a higher risk of infection or a more severe or more complicated course of the infectious disease. The determination of antibodies according to the present invention may also indicate additional treatment with antimycotics to reduce e.g. Candida, which would otherwise further spread and worsen the immune status of a given patient.

The present invention also relates to a method for prognosing the status of a patient who has undergone an organ transplantation, **characterised in that** the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level. A higher level of antibodies according to the present invention implies a higher risk of transplant rejection. This would indicate e.g. higher immune suppression medication from the very beginning.

The level of antibodies against TH17 immune response eliciting microorganisms may be determined by any method available in the art, such as ELISA-based methods, mass spectroscopy, etc. (see e.g. Current Protocols in Immunology; J. Wiley and Sons, Inc., 1991-2007).

The present invention, however, did not only reveal a diagnostic prognosis or disease status marker, but provides a novel target for these diseases. This is also a surprising explanation for clinical trials which have been conducted with an aggregated tumor antigen combined with Candida albicans antigens for the treatment of metastatic RCC. These trials failed; the vaccine had increased TH17 bias in these patients and thereby promoted tumor progression (Fowler, 1986). Accordingly, the present invention also provides a method for treating a tumor patient, a patient suffering from an autoimmune disease, a patient suffering from an infectious disease or a patient who has undergone an organ transplantation **characterised in that** an efficient amount of an agent which reduces the level of antibodies against TH17 immune response eliciting microorganisms is administered to said patient This may e.g. be performed by eliminating the antibodies with the isolated antigens (e.g. mannane-containing carbohydrate antigens, such as mannane, galactomannane or derivatives thereof). The specific elimination of the antibodies may preferably be combined with (unspecific) B cell depletion (with e.g. ritaximab).

TH17 immune response eliciting microorganisms are defined as microorganisms which elicit in a given host (especially in humans, which are, of course, the most preferred and most relevant patients according to the present invention) a TH17 biased immune response. Currently, it is known that Saccharomycetes, e.g. Saccharomycetaceae, especially Saccharomyces cerevisiae and Candida albicans, are yeasts/fungi which elicit TH17 biased immune responses and also infectious extracellular bacteria (Klebsiella pneumoniae; Ye et al, 2001). The present invention is, however, clearly not restricted to specific microorganisms, but is based on the fact provided with the present invention that TH17 immune responses have negative impact on tumor patients (Langowski et al., 2006 and 2007), autoimmune or transplant patients or patients with infectious diseases. The nature of TH17 immune responses is reviewed in the prior art e.g. in Steinman, 2007 and Acosta-Rodriguez et al., 2007 as well as in LeibundGut-Landmann et al., 2007.

Another aspect of the present invention is a direct consequence of the knowledge gained with the present invention: the fact that TH17 responses have a direct impact on the status of the patients, it is evident that in the field of B cell depletion treatments (e.g. in autoimmune diseases or leukaemias), the present invention is also directly applicable. Therefore, the present invention also relates to a method for monitoring a B cell depletion treatment comprising the detection of antibodies against TH17 immune response eliciting microorganisms at various time points of the treatment. During B cell depletion, the antibodies determined according to the present invention should decrase. This serves as a direct marker of the success of B cell depletion. At the same time, this also improves prognosis of this patient.

Directly connected with the methods described above is the need for providing antibodies against TH17 immune response eliciting microorganisms. Therefore, the present invention also provides a method for producing antibodies against TH17 immune response eliciting microorganisms with the following steps:
- isolation of B cells producing antibodies against TH17 immune response eliciting microorganisms;
- cultivation of the isolated B cells so as to obtain a B cell culture;
- inducing antibody production of said B cells in said B cell culture; and
- isolating the antibodies against TH17 immune response eliciting microorganisms from the B cell culture.

Methods for making human monoclonal antibodies from memory B cells are known in the art, e.g. from Traggiai et al., 2004. Therein, also an example for the isolation step is given: B cells are isolated immunomagnetically by CD22 microbeads; the B cells are then immortalised with EBV in the presence of CpG oligonucleotides; B cell clones are established by limited dilution and culture supernatants are screened for the presence of e.g. Saccharomyces or Candida IgG, i.e. by screening the supernatant with Saccharamoyces or Candida ELISAS.

Preferably, the cultivation is performed after infection of the B cells with Epstein-Barr virus (EBV), preferably in combination with an infection enhancing factor such as all cytokines of the Th17 family, preferably IL-21 and/or CD40 ligand, especially in combination with oligonucleotides containing a CpG motif.

Preferably, the induction is performed by addition of cytokines, especially interleukin 21 (IL-21) or, although with less efficiency, interleukin 2 (IL-2).. This is e.g. described in Kuchen et al., 2007 or Ettinger et al., 2005.

According to the present invention, antibodies against TH17 immune response eliciting microorganisms include all Ig isotypes (IgM, IgG1-4, IgA (IgE, to a less extent) which are directed against TH17 immune response eliciting fungi and preferentially members of the order Saccharomycetales such as but not restricted to Saccharomyces ssp. and Candida ssp. in patient fluids or tissues and to correlate the levels of such antibodies with the course of a disease. Furthermore, according to the present invention antibodies (all Ig isotypes) are measured which are directed against bacteria such as Klebsiella, which are known to induce TH17 type immune responses in patient fluids or tissues and to correlate the levels of such antibodies with the course of a disease.

According to the present invention it is possible to use antibodies directed against Saccharomyces, Candida, Aspergillus, Klebsiella (or other TH17-related organisms) as a prognostic indicator to predict the clinical course in tumors and other diseases such as infectious diseases, autoimmune diseases, atopic diseases or transplant rejection. Antibodies directed against Saccharomyces, Candida, Klebsiella (or other TH17-related organisms) may also be used as a serum marker of a bias of the immune system toward TH17 (serum marker of TH17 bias; according to Steinman, 2007).

According to the present invention also methods to inactivate existing B cells, which produce antibodies directed against Saccharomyces, Candida, Klebsiella or other TH17-related organisms are provided to positively influence the status or prognosis of the patients according to the present invention.

The present invention further provides methods to prevent binding of antibodies directed against Saccharomyces, Candida, Klebsiella or other TH17-related organisms to their corresponding antigens.

As mentioned above, the preferred fungi according to the present invention are fungi from the order Saccharomycetes (Kingdom: Fungi; Phylum: Ascomycota; Subphylum: Saccharomycotina; Class: Saccharomycetes). Especially the family of Saccharomycetaceae with the genera Ascobotryozyma, Candida (species: C. albicans, C. dubliniensis, C. glabrata, C. guilliermondii, C. kefyr, C. krusei, C. lusitaniae, C. milleri, C. oleophila, C. parapsilosis, C. tropicalis, and C. utilis), Citeromyces, Debaryomyces, Dekkera (Brettanomyces), Eremothecium, Issatchenkia, Kazachstania, Kluyveromyces, Kodamaea, Kregervanrija, Kuraishia, Lachancea, Lodderomyces, Nakaseomyces, Pachysolen, Pichia (Hansenula), Saccharomyces, Saturnispora, Tetrapisispora, Torulaspora, Vanderwaltozyma, Williopsis, and Zygosaccharomyces.

The invention is further described by the following example and the drawing figures, yet without being restricted thereto.

Fig. 1 shows a comparison of overall survival between patients with high or low serum levels of IgG against A) S. cerevisiae or B) cumulative bread components without S. cerevisiae; C) Comparison of overall survival between patients with high or low serum levels of hemoglobin;

Fig. 2 shows a comparison of overall survival between metastatic RCC patients with high or low serum levels of IgG against A) Candida ssp. or B) Bordetella pertussis or C) Mycoplasma pneumoniae.

### Example :

Autoantibodies against fungi as a prognostic indicator and a therapeutic target in cancer, infectious and autoimmune diseases as well as transplant rejection.

Experimental design: A commercial test was used to detect food-specific IgG directed against a panel of 113 food antigens in sera of 54 patients with metastatic RCC. Kaplan-Meier estimates were used for univariate survival analysis and differences in survival curves were assessed with the log-rank test. Multivariate survival analysis was done using a Cox regression model. Results: It was found that RCC patients with elevated serum levels of IgG antibodies against S. cerevisiae, commonly known as baker's yeast and yet another bread component, have an unfavourable clinical course. Median survival of patients with high levels of S. cerevisiae IgG was only 17.8 months, whereas median survival of patients with low S. cerevisiae IgG was 43.8 months (p = 0.0022; log-rank). Multivariate survival analysis identified high levels of S. cerevisiae IgG as a strong and independent prognostic risk factor (risk ratio 4.6, p=0.001; 95% CI 1.61-13.08).

Conclusions: These findings show that serum levels of IgG against S. cerevisiae predict survival in patients with metastatic RCC. The data suggest not cereals but baker's yeast being the critical component of bread, that may cause immune deviation and impaired immunosurveillance in predisposed RCC patients.

### Patients and methods

### Patients

54 RCC patients predominantly with clear-cell histology and bi-dimensionally measurable metastatic lesions were selected in a single-center study based on the availability of blood serum. No patient refused to enter the study. All patients were treated on consecutive IRB-approved dendritic cell vaccine trials, two of which have been published (Holtl et al., 2002, 2005). The primary tumor had been removed in all patients and the start of follow up was defined by the beginning of immunotherapy. Serum samples were collected prospectively prior to the onset of immunotherapy. Patients with solitary brain metastasis, other malignancies than RCC within the last 5 years, treatment with immunosuppressive drugs, other immunotherapies or chemotherapies within 4 weeks prior to treatment start, pregnancy or lactation, presence of acute or chronic infections, HIV or viral hepatitis or a Karnofsky index <60 were excluded from the study. Furthermore, a computed tomography (CT) of brain, chest and abdomen and a bone scan were performed. All patients were informed about the investigative character of the study and gave their written informed consent.

### Measurement of circulating anti-food IgG in patient serum

In the FoodSCAN Totality assay, patient serum samples were subjected to an enzyme-linked immunosorbent assay (ELISA) from YORKTEST Laboratories Ltd., Mils, Tirol, Austria, which uses microtiter plates coated with a panel of 113 different food antigens (Atkinson et al., 2004). Anti-food antibodies present in the patient's serum sample bind to the immobilized food antigen on the plate. A horseradish peroxidase-conjugated goat antihuman IgG was used to detect plate-bound food-specific patient IgG. Peroxidase-catalyzed color development using TMB as a substrate was subsequently measured using a visible light spectrophotometer (Molecular Devices E-Max Microtiter Plate Reader). Optical density (OD) at 450 nm is directly related to the concentration of anti-food IgG antibodies present in the sample. Test results were scored as positive or negative according to the manufacturer's cut-off definitions. In addition, the median IgG level was calculated (Table 2) to generate a high and a low level group and to perform survival analysis on the two groups.

### Survival Analysis

The endpoint of interest was survival time, defined as the time from treatment initiation to the date of death or the date of censure. For univariate analyses, Kaplan-Meier methodology was used to estimate survival distributions for bread components (n=5, Table 4) and clinical variables (Table 5). The relationship between survival and each of the variables was analyzed using the log-rank test.

A forward stepwise conditional Cox regression approach was used for the multivariate analyses, with 0.05 for entering and 0.1 for removing a variable in the model. Variables selected for the Cox model were age, sex, nuclear grade, serum IL-6, TNF-α, CRP, hemoglobin, lactate dehydrogenase (LDH) and S. cerevisiae IgG. The hazard ratios (risk ratios) and 95% confidence interval (CI) are reported. A two-sided p value of 0.05 or less was considered to indicate statistical significance. All calculations were carried out with SPSS software 13.0 (SPSS Inc., Chicago, Illinois).

### Results

Potential food intolerances identified by serum IgG testing were analyzed in 54 patients with metastatic RCC. Table 1 summarizes general patient characteristics and Table 2 shows median baseline laboratory parameters.

**Table 1. Patient characteristics**

| Characteristic | | No. | % |
|---|---|---|---|
| Age (years) | | | |
| Median | 58.0 | | |
| Range | 30 - 57 | | |
| Sex | | | |
| Female | | 14 | 25.9 |
| Male | | 40 | 74.1 |
| Median follow up (months) | 28.6 | | |
| Range | 5.4 - 90.5 | | |

| Pathologic stage | | | |
|---|---|---|---|
| M0 | | 5 | 9.3 |
| M1 | | 49 | 90.7 |
| | | | |
| N0 | | 29 | 53.7 |
| N1 | | 14 | 25.9 |
| N2 | | 9 | 16.7 |
| N3 | | 2 | 3.7 |
| | | | |
| T1 | | 9 | 16.7 |
| T2 | | 14 | 25.9 |
| T3a | | 15 | 27.8 |
| T3b | | 14 | 25.8 |
| T4 | | 1 | 1.9 |
| TX | | 1 | 1.9 |

| Histologic subtype | | | |
|---|---|---|---|
| Clear cell | | 50 | 92.6 |
| Clear cell with sarcomatoid proportion | | 3 | 5.6 |
| Papillary | | 1 | 1.9 |

**Table 2. Baseline laboratory parameters**

| **Laboratory serum parameter** | **Median** | **Range** |
|---|---|---|
| Protein (g/dl) | 7.2 | 6.2 - 8.5 |
| Albumin (mg/dl) | 4020 | 2300 - 5090 |
| Hemoglobin (g/l) | 133.0 | 82 - 160 |
| C-reactive protein (CRP) | 0.76 | 0.0 - 15.9 |
| Lactate dehydrogenase (U/ml) | 181.0 | 121 - 404 |
| IL-6 (pg/ml) | 8.0 | 0.0 - 463 |
| TNF-alpha (pg/ml) | 23.0 | 0.0 - 274 |
| Total calcium level (mmol/l) | 2.39 | 2.0 - 2.8 |

| **Serum IgG levels** | **Median** | **Range** |
|---|---|---|
| IgG against S. cerevisae (OD) | 0.200 | 0.074 - 1.158 |
| IgG against gluten (OD) | 0.082 | 0.054 - 1.047 |
| IgG against rye (OD) | 0.073 | 0.054 - 0.290 |
| IgG against wheat (OD) | 0.095 | 0.053 - 0.980 |
| IgG against spelt (OD) | 0.090 | 0.051 - 1.176 |

In the total study population, 49 of 54 patients (90.7%) had positive IgG testing to an average of 4 food antigens per patient (range: 1-18). RCC patient IgG were directed against 46 of the 113 different food antigens. Table 3 shows the distribution of food intolerances within the study population.

**Table 3. Frequencies of food - specific IgG (n=54)**

| Food | Frequency |
|---|---|
| S. cerevisiae | 29 (53.7%) |
| Cow's milk | 24 (44.4%) |
| Egg white | 17 (31.5%) |
| Egg yolk | 10 (18.5%) |
| Mussels | 10 (18.5%) |
| Beef | 8 (14.8%) |
| Goat's milk | 7 (13.0%) |
| Spelt | 7 (13.0%) |
| Wheat | 6(11.1%) |
| Millet | 5 (9.3%) |
| Kiwi | 5 (9.3%) |
| Garlic | 5 (9.3%) |
| Pde beans | 4 (7.4%) |
| Gluten | 3 (5.6%) |
| Hazelnut | 3 (5.6%) |
| Lamb | 3 (5.6%) |
| Cabbage mix | 3 (5.6%) |
| Melon mix | 3 (5.6%) |
| Cashew | 3 (5.6%) |
| Almond | 2 (3.7%) |
| Cowberry | 2 (3.7%) |
| Chili | 2 (3.7%) |
| Rye | 2 (3.7%) |
| Sde | 2 (3.7%) |
| Coconut | 2 (3.7%) |
| Orange | 2 (3.7%) |
| Kidney bean | 1 (1.9%) |
| Sunflower seed | 1 (1.9%) |
| Corn | 1 (1.9%) |
| Rice | 1 (1.9%) |
| Crustaceae | 1 (1.9%) |
| Withe fish mix | 1 (1.9%) |
| Aubergine | 1 (1.9%) |
| Cucumber | 1 (1.9%) |
| Lenses | 1 (1.9%) |
| Lettuce | 1 (1.9%) |
| Paprika | 1 (1.9%) |
| Soy beans | 1 (1.9%) |
| Ginger | 1 (1.9%) |
| Mustard | 1 (1.9%) |
| Sesame | 1 (1.9%) |
| Strawberry | 1 (1.9%) |
| Red currant | 1 (1.9%) |
| Cherry | 1 (1.9%) |
| Plum | 1 (1.9%) |
| Ananas | 1 (1.9%) |

| | |
|---|---|
| According to manufacturer's cut off | |

The most frequent intolerances were to S. cerevisiae (53.7%), cow's milk (44.4%), egg white (31.5%), egg yolk (18.5%), and mussels (18.5%) and thus similar although not identical in prevalence compared to other non-cancer patient study populations (Atkinson et al., 2004).

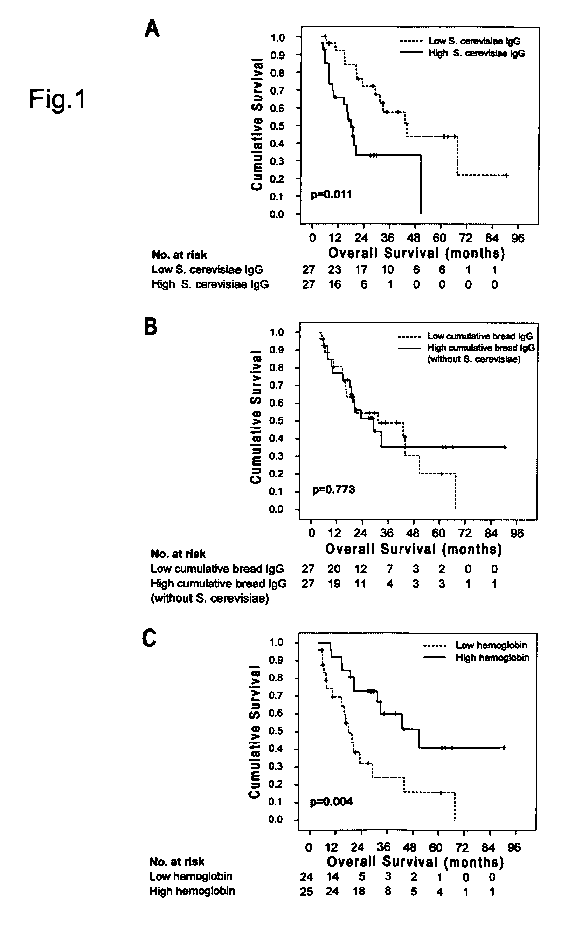

In the univariate survival analysis, however, patients with elevated serum levels (of IgG antibodies against S. cerevisiae, commonly known as baker's or brewer's yeast and yet another bread component, had an unfavourable clinical course (Fig. 1A and Table 4).

**Table 4. Univariate survival analysis of IgG against food antigens**

| Factor | Category | N | Median Survival | 95% - Confidence Intervall | P-value(log-rank) |
|---|---|---|---|---|---|
| S. cerevisiae IgG | Low | 27 | 43.8 | 25.2-62.4 | 0.011* |
| FoodSCAN Totality (OD) | High | 27 | 17.8 | 13.6-22.0 | |
| | | | | | |
| Gluten IgG | Low | 27 | 31.4 | 10.4-52.4 | 0.292 |
| | High | 27 | 29.1 | - | - |
| | | | | | |
| Rye IgG | Low | 27 | 42.9 | 0.6-85.1 | 0.917 |
| | High | 27 | 29.1 | | |
| | | | | 12.7-45.5 | |
| Wheat IgG | Low | 27 | 31.4 | 5.6-57.1 | 0.721 |
| | High | 27 | 29.1 | 16.0-42.1 | |
| | | | | | |
| Spelt IgG | Low | 27 | 42.9 | 7.1-78.6 | 0.929 |
| | High | 27 | 23.2 | 11.7-34.7 | |
| | | | | | |
| Cumulative bread IgG | Low | 27 | 31.4 | 5.6-57.2 | 0.773 |
| without S. cerevisiae | High | 27 | 29.1 | 13.0-45.2 | |
| | | | | | |
| Cumulative bread IgG | Low | 27 | 42.9 | 27.2-58.6 | 0.211 |
| with S. cerevisiae | High | 27 | 20.3 | 14.6-26.1 | |
| | | | | | |
| Food IgG | not present | 5 | all censured | all censured | 0.069 |
| | present | 49 | 23.2 | 11.7-34.6 | |
| | | | | | |
| All patients | | 54 | 31.4 | 16.3-46.5 | |

| | | | | | |
|---|---|---|---|---|---|
| * Bonferroni adjusted significance level for bread components; OD, optical density; | | | | | |

Median survival of patients with high levels of S. cerevisiae IgG was only 17.8 months, whereas median survival of patients with low S. cerevisiae IgG was 43.8 months (p = 0.0022; log-rank). The difference remains significant after α error adjustment according to Bonferroni (p = 0.011).

In contrast to IgG against S. cerevisiae, IgG antibodies against other bread components (gluten, rye, wheat and spelt) did not show a significant influence on patient survival although high levels were always accompanied by reduced survival (Table 4). Even cumulative values of IgG to all 4 bread components excluding S. cerevisiae IgG did not show a correlation with survival (Fig. 1B) with almost identical median survival times for the high and low IgG level group (29.1 and 31.4 months; Table 4). When cumulative values of IgG to all bread components that included S. cerevisiae IgG were analyzed, median survival for the high and low IgG group was 20.3 and 42.9 months, respectively (p=0.211; Table 4) showing that S. cerevisiae IgG and not IgG to other bread components correlate with patient survival. The well-established prognostic factor hemoglobin (Hb) showed the expected correlation with patient overall survival (Fig. 1C).

Of all 54 patients tested, 5 patients were completely negative for IgG to any of the 113 food antigens. Intriguingly, all 5 patients were alive at the time of analysis and absence of IgG to food antigens showed a tendency to correlate with prolonged survival (p=0.069) (Table 4).

Several serum markers have been established as prognostic risk factors in RCC. In the present study group hemoglobin (p=0.004), IL-6 (p=0.042) as well as TNF-α (p=0.032) correlated with patient survival and LDH showed a tendency to correlate with patient survival (p=0.092) (Table 5).

**Table 5. Univariate survival analysis of demographic and clinical variables**

| Factor | Category | N | Median Survival | 95%-Confidence Intervall | P-value (log-rank) |
|---|---|---|---|---|---|
| Age | <=58 | 27 | 29.1 31.4 | 14.4-43.8 | 0.808 |
| | >58 | 27 | | 1.9-60.9 | |
| | | | | | |
| Sex | Female | 14 | 32.7 | 10.2-55.2 | 0.873 |
| | Male | 40 | 23.2 | 6.6-39.8 | |
| | | | | | |
| Nuclear Grade | 1-2 | 29 | 31.4 | 10.6-52.2 | 0.186 |
| | 3-4 | 24 | 20.3 | 12.8-27.7 | |
| | | | | | |
| IL-6 (pg/ml) | Low | 25 | 42.9 | - | 0.042 |
| | High | 24 | 19.4 | 16.0-22.8 | |
| | | | | | |
| TNF-alpha(pg/ml) | Low | 24 | - | - | 0.032 |
| | High | 24 | 20.3 | 15.2-25.5 | |
| | | | | | |
| CRP (mg/dl) | Low | 26 | 31.4 | 20.8-42.0 | 0.604 |
| | High | 25 | 67.7 | 8.7.2- 126.6 | |
| | | | | | |
| Hemoglobin (g/L) | Low | 25 | 17.8 | 12.9-22.7 | 0.004 |
| | High | 26 | 50.7 | 25.5-75.8 | |
| | | | | | |
| Lactate dehydrogenase(U/mL) | Low | 25 | 42.9 | 27.9-57.9 | 0.092 |
| | High | 26 | 19.4 | 12.6-26.2 | |

| | | | | | |
|---|---|---|---|---|---|
| Abbreviations IL-6, interleukin-6; TNF-alpha, tumor-necrosis-factor-alpha; CRP, C-reactive protein; ASCA, anti-*S*. *cerevisiae* antibodies; | | | | | |

The variables included in the multivariate analysis were S. cerevisiae IgG plus the variables shown in Table 5. Using a significance level of 0.05 for entering and 0.1 for removing a variable in a forward stepwise analysis, high levels of S. cerevisiae IgG emerged as the strongest independent prognostic risk factor (risk ratio: 4.6, p=0.001; 95% CI 1.61-13.08). The two other variables that were retained, were TNF-α (risk ratio: 1.02, p=0.018; CI 1.01-1.04) and hemoglobin (risk ratio: 0,95 p=0.0001; CI 0.93-0.98)(Table 6).

**Table 6. Multivariate survival analysis**

| Factor | 95% Cl | Risk Ratio | P-value |
|---|---|---|---|
| S. cerevisiae IgG | 1.61 to 13.08 | 4.6 | 0.001 |
| TNF-alpha | 1.01 to 1.04 | 1.02 | 0.018 |
| Hemoglobin | 0.93 to 0.98 | 0.95 | 0.0001 |

No correlation was observed between serum levels of S. cerevisiae IgG and inflammation or infection markers such as CRP (r = -0.086, p = 0.546), TNF-α (r = 0.004, p = 0.977) or IL-6 (r = 0.008, p = 0.956) (Spearman-Rho). The current study included patients from two different study protocols using either autologous (Holtl et al., 2002) or allogeneic dendritic cells (Holtl et al., 2005). A current follow up revealed that the two study protocols resulted in very similar clinical outcomes with no significant difference in overall survival.

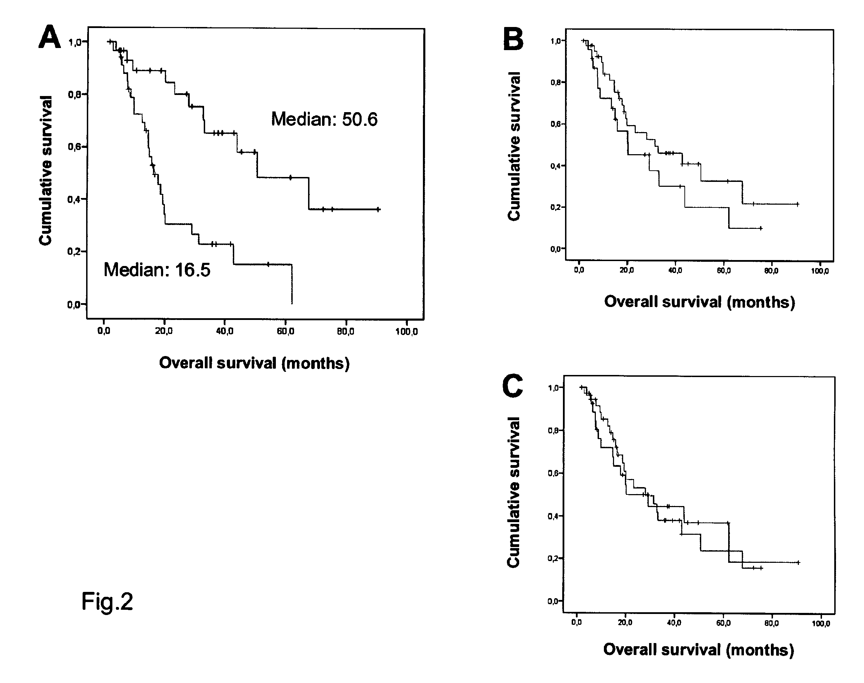

Similar results were obtained for C. albicans antibodies (Fig.2) Measurement of serum levels of IgG directed against a mixture of Candida species (C. albicans, C. krusei, C. glabrata, C. pseudotropicalis, C. parapsilosis in equal proportion) in 65 patients with metastatic RCC revealed an even more pronounced correlation between serum IgG levels and patient survival. Median survival of patients with high levels of Candida IgG was only 16.5 months, whereas median survival of patients with low Candida IgG was 50.6 months (p < 0.05; log-rank) (Fig. 2A). In contrast, no correlation was observed between serum levels of IgG specific for Bordetella pertussis (Fig. 2B) or serum levels of IgG specific for Mycoplasma pneumoniae (Fig. 2C).

### Discussion

IgG-based humoral immunity to food antigens in human metastatic RCC was evaluated and increased serum levels of S. cerevisiae IgG identified as a strong and independent prognostic risk factor. Patients with high levels of S. cerevisiae IgG had an increased risk of curtailed survival (Table 4 and 6). A correlation between CRP, TNF-α or IL-6 with serum levels of S. cerevisiae IgG was not observed indicating that IgG antibodies to S. cerevisiae are a stable marker, which is not regulated by inflammatory events or by infection and which is thus a useful independent prognostic indicator.

The presence of IgG antibodies is usually indicative of coexisting antigen-specific CD4+ T-cell immunity, because B cells require help from cognate CD4+ T helper (TH) cells for Ig class switching from IgM to IgG. The presence of IgG antibodies directed against S. cerevisiae in patient sera would thus be a predictor of the presence of CD4+ TH cells specific for S. cerevisiae. Intriguingly, CD4+ T-cell immunity against Candida albicans, a closely related yeast, is predominantly mediated by the TH17 subset of CD4+ T cells (Acosta-Rodriguez et al., 2007; LeibundGut-Landmann et al., 2007). TH17 cells, besides TH1 and TH2 cells, represent a third subset of polarized effector T cells. TH1 cells produce interferon-γ (IFN-γ) and confer immunity to viruses, intracellular bacteria and protozoan parasites. IFN-γ producing TH1 cells are also considered crucial for anti-tumor immunity. In contrast, TH2 cells making IL-4, IL-5 and IL-13 promote immunity against metazoan parasites. Immunopathology is often associated with TH2 cells since dysregulated TH2 cytokine production enhances IgE production, mucus secretion and eosinophilia thus favoring the development of allergic diseases such as asthma, allergic rhinitis, and atopic dermatitis.

TH17 cells characterized by the production of IL-17 and other cytokines appear to be required for resistance to infection by extracellular bacteria such as Klebsiella pneumoniae (Ye et al., 2001) as well as by fungi such as Candida albicans (Acosta-Rodriguez et al., 2007; LeibundGut-Landmann et al., 2007). Consistent with the assumption that serum IgG antibodies against S. cerevisae reflect TH17 differentiation, TH17 cytokine family members were shown to critically regulate inflammatory bowel diseases, such as Crohn's disease, where levels of IgG against S. cerevisiae are frequently increased (Barta et al., 2003; Bossuyt et al., 2006; Peeters et al., 2001), and TH17 T cells have been implicated in gut inflammation and destruction.

There is increasing evidence that TH1 (IFN-γ) and TH17 (IL-17) may be reciprocal in terms of function and that TH17 development is likely to occur at the expense of TH1, particularly because IL-23, which drives IL-17 producing TH17 cells, and IL-12, which promotes IFN-γ producing TH1 cells, share and thus compete for the common p40 subunit that heterodimerizes with p35 to form IL-12 or with p19 for IL-23 generation (Steinman, 2007). Enhanced TH17 development, however, may favor tumor development and progression (Langowski et al., 2006 and 2007). A role for IL-23 in promoting tumor incidence and growth was also described. Moreover, IL-23 stimulated angiogenesis and antagonized IL-12 and IFN-γ, both of which are crucial effector cytokines of antitumor immune responses.

Consistent with the notion that yeast-dependent TH17 bias are associated with an unfavorable clinical course of cancer, an early immunotherapy trial, which used an aggregated autologous tumor antigen combined with Candida albicans antigens as a nonspecific adjuvant for the treatment of metastatic RCC failed to demonstrate clinical activity (Fowler, 1986). Conversely, nine of 14 RCC patients receiving the Candida-containing tumor vaccine had disease progression manifested by the appearance of previously undetected metastases during the first 3 months. It is well possible that anti-Candida vaccination had increased TH17 bias in these patients and thereby promoted tumor progression.

Taken together, IgG antibodies directed against S. cerevisiae may be a serum marker of TH17 differentiation and the prognostic significance of such IgG antibodies in cancer patients may be due to the impaired immunosurveillance, which is a consequence of the immune deviating effects of TH17 cytokines that convert tumor-suppressing effector T cells into tumor-promoters.

The formation of large amounts of immune complexes consisting of food antigens and specific IgG antibodies on a daily basis may generally disturb immunosurveillance. Antigen-antibody complexes have been shown to inhibit IL-12 production and to induce IL-6 and prostaglandins thereby generating a milieu, which enhances TH17 differentiation. In line with the assumption that immune complexes cause immunosuppression or immune deviation and thus disturb cancer immunosurveillance is the intriguing although preliminary finding in our study that the 5 patients, who had no detectable IgG against the whole panel of 113 food antigens, were all alive at the time of analysis and absence of IgG to food antigens showed a tendency to correlate with prolonged survival (p=0.069) (Table 4). Moreover, one of these 5 patients had a complete remission of all metastases during a recent immunotherapy trial with dendritic cells and is still tumor free after 2 years.

In conclusion, the study according to the present invention identifies S. cerevisiae as the critical component of bread, which has recently been shown to have an unfavorable role in the development of RCC.

### References:

Acosta-Rodriguez et al., (2007) Nat Immunol 8:942-949
Acosta-Rodriguez et al., (2007) Nat Immunol 8:639-646
Atkinson et al., (2004) Gut 53:1459-1464
Barta et al., (2003) World J Gastroenterol 9:2308-2312
Bossuyt et al., (2006) Clin Chem 52:171-181
Bravi et al., (2007) Int J Cancer 120:681-685
Ettinger et al., (2005) J Immunol 175: 7867-7879
Fowler (1986) J Urol 135:22-25
Holtl et al., (2005) Cancer Immunol Immunother 54:663-670
Holtl et al., (2002) Clin Cancer Res 8:3369-3376
Kuchen et al., (2007) J Immunol 179 5886-5896
Langowski et al. (2006), Nature 442: 461-465
Langowksi et al. (2007), Trends Immunol. 28: 207-212
LeibundGut-Landmann et al. (2007), Nat. Immunol. 8: 630-638
Peeters et al., (2001) Am J Gastroenterol 96:730-734
Steinman (2007) Nat Med 13:139-145
Traggiai et al., (2004) Nat Med 10: 871-876
Ye et al., (2001) J Exp Med 194:519-527

## Claims

1. Method for prognosing the status of a tumor patient, **characterised in that** the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level.

2. Method for prognosing the status of a patient suffering from an autoimmune disease, **characterised in that** the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level.

3. Method for prognosing the status of a patient suffering from an infectious disease, **characterised in that** the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level.

4. Method for prognosing the status of a patient who has undergone an organ transplantation, **characterised in that** the level of antibodies against TH17 immune response eliciting microorganisms is determined in said patient, and prognosing the status of the patient upon the level of these antibodies determined in the patient by determining a better prognosis for a patient with a lower level of these antibodies compared to the average level or by determining a worse prognosis for a patient with a higher level of these antibodies compared to the average level.

5. Method for treating a tumor patient, a patient suffering from an autoimmune disease, a patient suffering from an infectious disease or a patient who has undergone an organ transplantation **characterised in that** an efficient amount of an agent which reduces the level of antibodies against TH17 immune response eliciting microorganisms is administered to said patient

6. A method according to any one of claims 1 to 5 **characterised in that** said TH17 immune response eliciting microorganisms are selected from Saccharomycetes, preferably Saccharomycetaceae, especially Saccharomyces cerevisiae and Candida albicans, and Klebsiella pneumoniae.

7. : A method for monitoring a B cell depletion treatment comprising the detection of antibodies against TH17 immune response eliciting microorganisms at various time points of the treatment.

8. Method for producing antibodies against TH17 immune response eliciting microorganisms **characterised in** the following steps:
- isolation of B cells producing antibodies against TH17 immune response eliciting microorganisms ;
- cultivation of the isolated B cells so as to obtain a B cell culture;
- inducing antibody production of said B cells in said B cell culture; and
- isolating the antibodies against TH17 immune response eliciting microorganisms from the B cell culture.

9. Method according to claim 8, **characterised in that** said cultivation is performed after infection of the B cells with Epstein-Barr virus (EBV), preferably in combination with an infection enhancing factor, especially in combination with oligonucleotides containing a CpG motif.

10. Method according to claim 8 or 9, **characterised in that** said induction is performed by addition of cytokines, especially interleukin 21 (IL-21).
